# EUROPEAN PATENT SPECIFICATION

(11) **EP 3 876 240 B1**
(45) Date of publication and mention of the grant of the patent: **05.07.2023**
(21) Application number: 20161541.6
(22) Date of filing: 06.03.2020
(51) Int. Cl.: G16H 50/50, A61B 5/00, G06F 3/01, G09B 9/00, G09B 19/00, A61B 17/08, A61B 34/10, A61F 13/00, A61L 15/16, A61M 1/00, A61M 5/00

(54) **VIRTUAL REALITY-BASED TRAINING PROGRAM FOR A WOUND CARE PROFESSIONAL**
VIRTUELLE REALITÄT BASIERTE AUSBILDUNGSPROGRAM FÜR WUNDBEHANDLUNGSFACHKRÄFTE
PROGRAMME DE BASE RÉALITÉ VIRTUELLE POUR LES PROFESSIONELLES QUI SOIGNENT DES BLESSURES

(43) Date of publication of application: 08.09.2021
(73) Proprietor: Paul Hartmann AG, 89522 Heidenheim (DE)
(72) Inventor: SCHUCK, Peter, 89520 Heidenheim (DE); BORDEANU, Adriana-Luiza, 89284 Pfaffenhofen a. d. Roth (DE); CSERNUS, Mariann, 89518 Heidenheim (DE); BLOCH, Christine, 97353 Wiesentheid (DE)

(56) References cited:
- US-A1- 2008 187 896
- US-A1- 2012 270 196
- US-A1- 2019 057 620

## Description

The present invention relates to a method and a technical system which enables interactive training of a wound care professional in a virtual reality (VR) environment.

The present subject matter further relates to a computer program for interactive training of a wound care professional in a VR environment and a computer-readable storage device upon which the program is saved.

### Background

A wound can be regarded as separation of the contiguity of tissues of the skin, wherein this can be combined with a loss of substance.

The healing of wounds is based on the ability of the skin to regenerate tissue such as epithelial tissue, connective and supporting tissue. The regeneration of tissue is a complex occurrence of cell activities overlapping with each other, wherein said cell activities promote the healing process step by step.

Professional wound treatment includes i.a. correct diagnosis, selection of a suitable treatment method, manual treatment of the wound if necessary, such as irrigation, selection of suitable dressings and further observation of the healing process. The successful treatment of wounds relies on health personnel, in particular wound care professionals, having both in-depth knowledge and practical experience obtained from a large number of patients and wound treatment cases.

The supervised wound healing process can last from several days up to up several weeks or months. The wound healing process may also stagnate resulting in a chronic wound status. In order to improve his practical skills, a wound care professional must accompany the entire process so that he can directly observe the effect of the wound treatment methods he has chosen.

However, the number of patients with wounds in a medical facility is limited and not every type of wound is always within reach for a wound care professional.

In addition to practical training of wound care professionals, medical knowledge may be taught either in a classroom or by self-study using books or online resources.

Recently, is has also become possible to apply VR methods to convey specialist knowledge and skills in the medical field as well.

An example of a medical training software is presented in CN106295198 which provides standardized patient types and displays the symptoms of various diseases to the user. The user interacts with the virtual patient and makes a diagnosis. The system then produces evaluation of the user interaction with the system.

US2012270196 A1 may be considered to disclose a computer-implemented method for the training of a user, who is a wound care professional, in a virtual environment comprising the steps:
a. Displaying a first interactive scene, which represents a medical scenario comprising an image of a wound;
b. Providing the user with multiple wound treatment options;
c. Receiving and processing a user input corresponding to one or more wound treatment selections performed by the user, whereby each wound treatment selection is based on one of the wound treatment options;
d. Evaluating the user input and the gauge panel;
e. Displaying a further scene updated in relation to the wound healing process, whereby the updating is based on the evaluation of the user input,
so that the user can experience a realistic wound healing process in time-lapse.

### Detailed description

The objective of the present invention was to improve the treatment of patients having severe and/or chronic wounds. In particular the objective was to improve the treatment-skills of wound care professionals.

The problem is solved by a computer-implemented method for the training of a wound care professional in a VR environment as presented in claim 1, a system for the training of a wound care professional in a VR environment as presented in claim 12, a computer program which enables the execution of the method for the training of a wound care professional in a VR environment and the computer-readable storage device having embodied thereon the before mentioned computer program as presented in claim 16 and 17, respectively.

The invention concerns a computer-implemented method for the training of a wound care professional using a VR environment. The wound care professional may be a medical doctor or a nurse, for example. Herein, the wound care professional is also referred to as the "user". The inventive method comprises the steps:
a. The display of a 3D interactive scene, which represents a medical scenario comprising the image of a wound. As referred herein, the displaying includes all computing steps that must be carried out to obtain a 3D scene. These steps may include but are not limited to modeling, simulation or rendering. Displaying of the 3D interactive scene by the technical means of VR provides a more immersive experience than the mere presentation of wound images in textbooks or a computer screen. For example, it is possible to examine a virtual patient, who has a virtual wound, from different angles. This virtual interaction with the virtual patient in a VR environment realistically mimics the daily practice of a wound care professional. In the context of the present application the term "medical scenario" describes any scene, which a medical professional may encounter in real life, for example a hospital room or an accident scenario.
b. Further, the method provides the user with multiple wound treatment options. Any kind of medical interventions that can help to enable or accelerate wound healing of any arbitrary wound type can be considered as a wound treatment option. The treatment options may include, for example, the application of dressing materials, debridement and/or cleaning procedures, negative pressure wound treatment, plasma wound treatment, application of light and/or application of active substances to the wound.
c. The user can choose from one or more of the treatment options resulting in one or more treatment selections performed by the user, whereby each wound treatment selection is based on one of the wound treatment options. In the VR environment the selection can be signaled to the VR system (a user input), for example, by pointing with the virtual finger at a dressing or at a medical device. It would also be possible, as another non-limiting example, to grip the product which is required for the selected treatment option by the virtual hand and apply it to the virtual wound. The user input corresponding to one or more wound treatment selections is received and processed by the virtual scene building unit.
d. The method further includes an evaluation of the user input. Evaluation can be performed after the user finalizes the virtual treatment, which comprises the treatment selection and optionally an application of a product to the wound.
e. The method further includes a subsequent 3D interactive scene updated in relation to the wound healing process, whereby the updating is based on the evaluation of the user input. The term "updating is based on the evaluation of user input" means that the VR system evaluates the user input received in step c and thereafter simulates that a virtual wound shown in step a is treated with the one or more treatment options selected by the user. This simulation leads to the display of a further 3D interactive scene in the VR environment. At this step the user experiences the consequences of the selected treatment comparable to a real-life wound treatment situation.

The proposed VR based training uses technical means that allow the wound care professional to experience a realistic wound healing process in time-lapse. Conveyed by the technical means of a VR system the wound care professional has a cognitive experience as if he had treated a real patient and he can directly observe the consequences of his decisions. Furthermore, the VR setting allows for a risk-free environment in which wrong decisions are allowed and training experiences can be repeated if necessary. The technical means used for this invention allow for a synergistical combination of the advantages of instructor (or media-based) training approaches and practical training.

According to a preferred embodiment the image of a wound is a 3D image. The 3D image provides the user a much more realistic representation and the user is able to observe and examine the wound from different angles.

The proposed method comprises a first medical scenario where the scenario may further comprise one or more of the elements selected from a patient who displays the wound, a hospital bed, a selection of wound treatment options and a panoramic virtual scene. The panoramic virtual scene can reproduce, for example, a typical hospital environment.

The method comprises displaying a wound image, preferably a 3D Image, as a component of the medical scenario. The wound image can be authentic pictures of a wounds of a real patient (a "real-life wound"). The image may also be a modification of pictures taken from a wound of a real patient. They may as well be artistic representations of wounds based on medical knowledge about wounds.

In a preferred embodiment the wound image is generated from real-life patient image data. According to this preferred embodiment, only one wound image of a single patient is used to generate the image in VR. In another preferred embodiment multiple images of one wound may be used to reconstruct a more vivid image, in particular a more vivid 3D image. In yet another embodiment the real-life patient image data of one or multiple patients may be used to generate the VR representation of the wound. The image of the wound (in particular a 3D image), may also be realized by employing machine learning. The dataset of real patient data may then be used as training data.

Further, the method provides the user with multiple wound treatment options. Those treatment options may include the selection of different wound treatment products such as different types of dressings like gauze dressings, hydrogel dressings or foam dressings. The treatment options may also include the application of wound treatment devices such as negative pressure devices or plasma generating devices.

According to a preferred embodiment the user can observe a correct execution of the one or more wound treatment options (selected by the user) in the VR environment. The VR system may generate a dynamic VR scenario, for example by displaying a short VR simulation. The execution of an option is for example the application of a wound dressing or the carrying out of a negative pressure wound therapy.

The method of the invention simulates and illustrates the progression of the wound over time. The wound healing process is shown after a period of 1 minute to 90 days, preferably after a period of 1 hour to 60 days, more preferably after a period of 1 hour to 28 days, in particular after a period of 1 hour to 7 days or after a period of 1 to 3 days. The user experiences the consequences of the selected treatment immediately instead of having to wait for an extended period of time as would be the case in a real-life treatment situation.

Optionally, the method allows for additional feedback or information concerning the one or more treatment options as selected by the user to be relayed to the user. This additional feedback can go beyond that of the updated VR scenario including the wound progression. According to a preferred embodiment, the feedback is one or more text blocks displayed in the VR environment. It may also be an audio-feedback, such as the voice of an instructor.

The inventive method allows for a plurality of first 3D interactive scenes as given by step a of the proposed method. Those scenes may differ in one or more of the following non-limiting characteristics: wound type, severity of wound, characteristics of the patient or any other parameters. A possibility to determine the first 3D interactive scene is by selection of the user or by randomization. In contrast to a real-life hospital (i.e. a hospital existing in reality), where only patients having a limited number of different wound types are present, the variety of the interactive VR scenarios may provide a wound care professional with a wider range of wound types.

Each step a to e of the method for the training of wound care professionals in a VR environment as given by claim 1 has to be executed at least once and in the given order. According to a preferred embodiment, the method contains one or more a repetitions of steps b to e. In a preferred embodiment the steps b to e are carried out once or several times until a termination condition is met. The termination condition may be a cured wound, the user exiting the program or a decision that leads to irremediable consequences for the virtual patient like an amputation. According to a further embodiment, upon selecting the wrong treatment option, a fallback to a previous state of the wound is possible as long as the termination condition is not met. In another embodiment the computer-generated regression of the wound may be shown. While the consequences of wrong decisions are experienced by the user more realistic in a VR environment compared to a digital or printed 2D-reproduction of the wound, any misdiagnosis does not cause harm to real patients.

The invention further pertains to a technical system for the training of a wound care professional in a VR environment comprising a VR imaging unit to acquire a 3D image, at least one input device, a virtual scene building unit, a data storage unit and an executable software adapted to perform the following steps:
a. Displaying a first 3D interactive scene, which represents a medical scenario comprising the image of a wound, on the VR imaging unit by means of the virtual scene building unit
b. Providing the user with multiple wound treatment options displayed on the VR imaging unit and selectable by means of the input device
c. Receiving and processing a user input corresponding to one or more wound treatment selections performed by the user, whereby each wound treatment selection is based on one of the wound treatment options by means of the virtual scene building unit
d. Evaluating the user input on the virtual scene building unit
e. Displaying a further 3D interactive scene updated in relation to the wound healing process, whereby the updating is based on the evaluation of the user input, on the VR imaging unit by means of the virtual scene building unit.

The VR imaging unit is any apparatus that allows the user to experience a VR environment.

The technical system has at least one input device. An input device can be any device that enables the VR imaging unit to receive signals and data from the environment. The environment includes the user. An embodiment of the VR scene building unit comprises a computer system, which can perform the calculations and computing steps necessary to obtain a 3D image in the VR environment. The VR scene building unit may include a database with which the computer system communicates.

The technical system comprises a computer system which possesses at least on processing unit. A processing unit can be a CPU (central processing unit) but also a GPU (graphics processing unit). The computer systems, which meet this requirement, may be a desktop computer, a mobile computer or a game console.

The technical system comprises a VR imaging unit which is a head-mounted display, for example VR glasses.

The technical system comprises at least one input device which may be realized by one or more of the following devices: wireless, hand-held pair of 3D motion controllers, VR gloves or full body reading systems. But also motion sensors in VR headsets or a keyboard connected to the computer system to initiate the computer program may be considered as additional input devices.

Receiving the user input in step c of the method comprises any kind of user input corresponding to one or more wound treatment selections performed by the user. The user input may also include a combination of signals from different input sources, in particular a combination of signals from the different input devices mentioned herein.

The invention also concerns a computer program, which enables the execution of the method for the training of a wound care professionals in a VR environment.

Moreover, the invention pertains to a computer-readable storage device having embodied thereon a computer program for executing the method for the training of a wound care professional in a VR environment.

### Short description of the figures

For a better understanding of the present invention and its advantages, reference is now made to the following embodiments in conjunction with the associated figures.

In the following, the invention is explained in more detail by means of exemplary embodiments which are indicated in the schematic illustrations of the figures.

The figures show:
Fig. 1: a schematic diagram of the system for the training of a wound care professional in a VR environment according to a preferred embodiment of the invention.
Fig. 2: a flow chart of the computer-implemented method for the training of a wound care professional in a VR environment according to a preferred embodiment of the invention.

### Detailed description of the figures

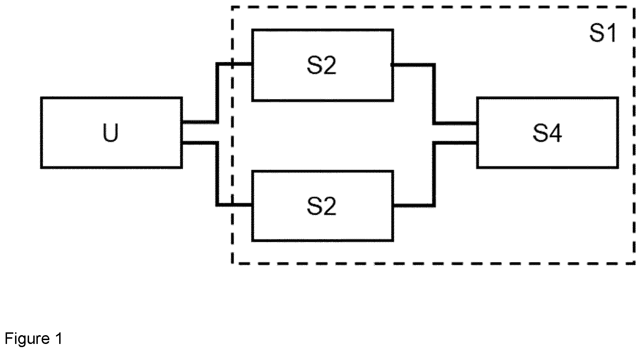

Figure 1 shows a schematic diagram of the system for the training of wound care professionals in a VR environment according to a preferred embodiment of the invention.

The technical system S1 comprises the VR imaging unit S2, at least one input device S3 and the virtual scene building unit S4. The user is depicted as U.

The VR imaging unit S2 is any device or system of devices capable of letting the user experience a VR environment. In a preferred embodiment the VR imaging unit S2 are head-mounted VR glasses, such as for example the Oculus Rift S, provided by Facebook Technologies, LLC or the HTC Vive head-mounted display by HTC and Valve. Other examples for head-mounted VR glasses are Zeiss VR One or Zeiss VR One Plus by Carl Zeiss AG, Gear VR by Samsung Group, LG 360 VR by LG Group or Playstation VR by Sony Corporation. For a cheaper solution, the combination of a smartphone and a mounting device with lenses is possible. The smartphone display is split into two parts by means of an app and performs the calculations for a stereoscopic view. An example for such a mounting device is Google Cardboard.

The input device S3 enables the user U to interact with the scene building unit S4. The input device S3 also captures sensor input to the be transmitted to the scene building unit S4. There is at least one input device S3 in the technical system.

The virtual scene building unit S4 comprises a computer system which possesses at least one processing unit. A processing unit can be a CPU but also a GPU. The computer system may also have access to a database, which may be held on an external server connected to the computer system using for example radio communication like a Wi-Fi connection.

The technical system S1 interacts with the user U by means of the virtual scene building unit S4 and the at least one input device S3. Data is relayed between the virtual scene building unit S4 and the VR imaging unit S2 and between the virtual scene building unit S4 and the input device S3.

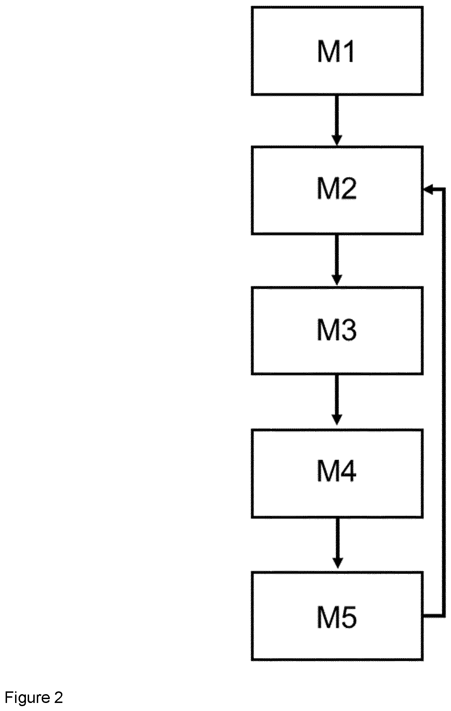

Figure 2 shows a flow chart of a computer-implemented method for the training of a wound care professional in a VR environment according to a preferred embodiment of the invention.

The method comprises an initial step M1. Step M1 comprises displaying a first 3D interactive scene which represents a medical scenario comprising the image of a wound. The first scene may also comprise for example a patient, a hospital bed and a panoramic view. The wound image may be generated from real-life patient data. The necessary calculations for displaying the initial scene are performed on the virtual scene building unit S4 and displayed on the VR imaging unit S2 of the technical system S1.

In step M2 of the method the user U is provided with multiple virtual wound treatment options. The wound treatment options may for example include the application of wound treatment products or wound treatment apparatuses. The visualization of the wound treatment options may for example be the display of different types of wound dressings laying and/or wound treatment apparatuses on a side table. The wound treatment options are displayed in the VR environment by the VR imaging unit S2 and can be selected by the user U by means of the one or more input devices S3. In a preferred embodiment the input device S3 comprises a wireless, hand-held, two-piece, 3D motion controller. An example of this controller can be the HTC Vive Controller by HTC and Valve or Oculus Touch by Facebook Technologies, LLC.

In step M3 of the method the user U chooses one or more wound treatment options resulting in one or more treatment selections. In a preferred embodiment the correct application of the wound treatment as selected by the user U is demonstrated by the VR system. The user U is given a visual manual on how to use the wound care product or the wound care devices. After the selection of the wound treatment option, the user U confirms the selection of the treatment options and initiates the evaluation of his treatment selection by means of the input device S3.

The user input from the one or more input devices S3 is received and processed. This task is executed by the virtual scene building unit S4.

After receiving and processing the user input, the virtual scene building unit S4 evaluates the effects of the selected wound treatment option on wound healing in step M4 and scene building unit S4 determines the selection of the new scene. The virtual scene building unit S4 may compare the selection of the user against a decision tree saved locally or provided by a database. These decision trees can be created and, if necessary, extended by medical professionals or they can be created by machine learning, where a statistical model is trained with data gathered from real wound treatments.

The method comprises the display of the subsequent scene M5, in which the virtual wound is shown in an appearance that simulates further progression of the treatment process for a certain period of time and under the impact of the one or more wound treatment selections by the user U. The wound healing process is shown after a period of 1 minute to 90 days, preferably after a period of 1 hour to 60 days, more preferably after a period of 1 hour to 28 days, in particular after a period of1 hour to 7 days or after a period of 1 hour to 3 days. Depending on the user selection in step M3 the wound may be displayed as if healing has further advanced, as if it had undergone a deterioration of the status or if it had been unaffected by said virtual treatment. The presentation of the healing process may also include the wound environment, the general health and thus the appearance of the patient, as well as the appearance of the removed wound dressing or bedding. The scene is displayed on the VR imaging unit S2 based on calculations from the virtual scene building unit S4 in the previous step.

The steps M1 to M5 are executed at least once in each training circle. In a preferred embodiment the steps M2 to M5 may also be repeated. The repetition of steps M2 to M5 continues until one of the possible the exit conditions is met. The exit conditions comprise a cured wound in step M5, the user U exiting the program or the user selecting a wound treatment option which leads to irremediable consequences for the virtual patient like an amputation. After the exit of a scenario, it is possible that the program saves a score corresponding to the user's performance during the scenario. The score may be limited to one scenario or be cumulative over multiple scenarios. The score may be personalized to reflect the performance or improvement of skill of an individual wound care professional.

In one embodiment the initial scene M1 is chosen by the user U from a plurality of possible initial scenes. In another embodiment the initial scene is randomly selected from a plurality of possible initial scenes. It is conceivable that a distinction is made between categories such as "beginner" and "advanced". This distinction can be determined by the user's decision or by the program, taking into account whether the user U has encountered this scenario before and how successful he has been in learning.

The inventive method is performed by a computer program when the computer program is executed on the computer system of the virtual scene building unit S4. The computer program is further stored on a computer-readable storage device, for example a solid-state drive or a hard disk drive.

Although specific embodiments have been illustrated and described herein, it is understandable to the expert that a variety of alternative and/or equivalent implementations exist. It should be noted that the exemplary embodiment or embodiments are only examples and are not intended to limit the scope, applicability or configuration in any way.

Rather, the summary and detailed description referred to above provides the expert with a convenient guide to the implementation of at least one exemplary embodiment, it being understood that various changes in the functional scope and arrangement of the elements can be made without deviating from the scope of the attached claims.

### Example

The user U starts the wound care training program on the virtual scene building unit S4, which is a portable computer, and puts on the VR headset, which is the VR imaging unit S2. The user U now experiences the virtual environment. The user U controls the interaction with the virtual environment by means of a wireless, two-piece controller in both hands and by motion sensors in the VR headset. Movement of the head of the user U is detected by said motions sensors and also leads to a movement of the user U in the VR environment including a change of his fields of vision. Accordingly, the computer system S4 receives input from both the VR controllers and the VR headset

The VR headset is also the main output device of the computer system. It enables the user U to experience VR. However, since it also comprises motion sensors which send signal data to the computing unit, the VR headset is the VR imaging unit S2 as well as an input device S3.

In the first scenario M1 in VR environment the user U is placed in a hospital room with a patient on a bed. The patient features a wound on his leg. The wound image is generated from a photograph of a real-life wound. The VR environment further shows a table located next to the bed. The user U will find a plurality of wound care products on top of the table. In this example the plurality of wound care products constitutes the multiple wound treatment options (scenario in step M2). In step M3 the user U can select one of the provided wound care products, for example a foam dressing by means of hand motion and hand gestures recorded by the VR controller. The user U may also "apply" the selected dressing by gripping it and moving it close to the virtual wound. According to a preferred embodiment, the user can then observe correct application of the wound dressing., which means that the correct application of the wound care product is demonstrated in the VR environment. In an even more preferred embodiment, the virtual body of the user or parts thereof, such as the virtual hands, perform the application of the wound care product. Hereby the user U gets a training experience as if the application has been performed by himself. During this time, the user U cannot control the virtual body and, therefore, cannot make another treatment selection. The user U is now able to select and apply additional wound care products to the wound. Each selected wound care product is preferably followed by a short 3D animation of its correct application, as explained above. The user U confirms his treatment selection and initializes the evaluation process by pressing a button next to the wound care options.

The virtual scene building unit S4 evaluates the treatment selection of the user U in step M4. The VR system may provide feedback to the user U, for example by means of a text block floating in the VR environment to the user U. The text block is displayed to the user U in his field of vision. An example for the contents of the text block is an evaluation of the selected treatment option. The virtual scene is then updated in relation to the wound healing process based on the evaluation of the user input resulting in the display of a further interactive 3D scene M5. If the treatment choice was adequate, the subsequent scene M5 features a wound having an appearance corresponding to an improved healing status. In the case of an inappropriate or adverse treatment choice the wound deteriorates back to its initial condition. The steps following the wound treatment selection from the provided wound treatment choices M2 up to the updated 3D scene M5 are repeated until the user U chooses the correct treatment option for each wound healing step. Then the wound appears as healed in the final instance of the VR environment.

The user U exits the VR environment.

## Claims

1. A computer-implemented method for the training of a user (U), who is a wound care professional, in a VR environment comprising the steps:
a. Displaying a first 3D interactive scene (M1) by the technical means of virtual reality (VR), which represents a medical scenario comprising an image of a wound, whereby the technical means of virtual reality (VR) include a head-mounted display,
b. Providing the user (U) with multiple wound treatment options (M2)
c. Receiving and processing a user input (M3) corresponding to one or more wound treatment selections performed by the user (U), whereby each wound treatment selection is based on one of the wound treatment options
d. Evaluating the user input (M4)
e. Displaying a further 3D interactive scene (M5) updated in relation to the wound healing process, whereby the updating is based on the evaluation of the user input,
so that the user (U) can experience a realistic wound healing process in time-lapse.

2. The method according to claim 1, wherein the image of a wound is a 3D image.

3. The method according to claim 1 or 2, wherein the medical scenario further comprises one or more of virtual elements selected from a patient who displays the wound, a hospital bed, a selection of wound treatment products, a selection of wound treatment devices and a panoramic virtual scene.

4. The method according to one or more of the preceding claims, wherein the image of a wound is generated from real-life patient data.

5. The method according to one or more of the preceding claims, wherein the image of the wound is either generated from a single image of a real-life wound or generated from multiple images of real-life wounds, for example by means of machine learning.

6. The method according to one or more of the preceding claims, wherein the wound treatment options are wound treatment products, in particular wound dressings, or wound treatment devices.

7. The method according to one or more of the preceding claims, wherein the user (U) can observe a correct execution of the one or more selected wound treatment options in the VR environment.

8. The method according to one or more of the preceding claims, wherein the wound healing process is shown after a period of 1 minute to 90 days, preferably after a period of 1 hour to 60 days, more preferably after a period of 1 hour to 28 days, in particular after a period of 1 hour to 7 days or after a period of 1 hour to 3 days.

9. The method according to one or more of the preceding claims, wherein the user (U) receives additional feedback or information concerning the treatment selection in the virtual reality (VR) environment

10. The method according to one or more of the preceding claims, wherein the steps a to e are executed at least once and wherein the steps b to e are repeated until a termination condition is met.

11. The method according to one or more of the preceding claims, wherein the first 3D interactive scene is selected from a plurality of 3D interactive scene options either by a user selection or at random.

12. A technical system (S1) for the training of a user (U), who is a wound care professional, in a virtual reality (VR) environment, comprising a virtual reality (VR) imaging unit (S2), wherein the virtual reality (VR) imaging unit (S2) is a head-mounted display, to acquire a 3D image, at least one input device (S3), a virtual scene building unit (S4), a data storage unit and an executable software adapted to perform the following steps:
a. Displaying a first 3D interactive scene (M1), which represents a medical scenario comprising an image of a wound, on the virtual reality (VR) imaging unit (S2) by means of the virtual scene building unit (S4)
b. Providing the user (U) with multiple wound treatment options (M2) displayed on the virtual reality (VR) imaging unit (S2) and selectable by means of the input device (S3)
c. Receiving and processing a user (U) input (M3) corresponding to one or more wound treatment selections performed by the user (U), whereby each wound treatment selection is based on one of the wound treatment options, by means of the virtual scene building unit (S4)
d. Evaluating the user input (M4) on the virtual scene building unit (S4)
e. Displaying a further 3D interactive scene (M5) updated in relation to the wound healing process, whereby the updating is based on the evaluation of the user input, on the virtual reality (VR) imaging unit (S2) by means of the virtual scene building unit (S4), so that the user (U) can experience a realistic wound healing process in time-lapse.

13. The system (S1) according to claim 12, wherein the virtual scene building unit (S4) comprises a computing device comprising one or more processing units, such as a CPU or a GPU.

14. The system (S1) according to claim 12 or claim 13, wherein each of the at least one input devices (S3) is a virtual reality (VR) controller, in particular a wireless, hand-held, two-piece, 3D motion virtual reality (VR) controller, one or more virtual reality (VR) gloves and/or a full body reading system.

15. A computer program which enables the execution of the method for the training of a user (U), who is a wound care professional, in a virtual reality (VR) environment presented in one or more of the claims 1 to 11.

16. A computer-readable storage device having embodied thereon a computer program for executing the method for the training of a user (U), who is a wound care professional, in a virtual reality (VR) environment presented in one of the claims 1 to 11.

## Patentansprüche

1. Computerimplementiertes Verfahren für das Trainieren eines Benutzers (U), der eine Wundversorgungsfachkraft ist, in einer VR-Umgebung, umfassend die Schritte:
a. Anzeigen, durch das technische Mittel einer virtuellen Realität (Virtual Reality, VR), einer ersten interaktiven 3D-Szene (M1), die ein medizinisches Szenario darstellt, umfassend ein Bild einer Wunde, wobei das technische Mittel der virtuellen Realität (Virtual Reality, VR) eine kopfmontierte Anzeige beinhaltet,
b. Bereitstellen mehrerer Wundbehandlungsoptionen (M2) für den Benutzer (U)
c. Empfangen und Verarbeiten einer Benutzereingabe (M3), die einer oder mehreren Wundbehandlungsauswahlen entspricht, welche von dem Benutzer (U) durchgeführt werden, wobei jede Wundbehandlungsauswahl auf einer der Wundbehandlungsoptionen basiert
d. Auswerten der Benutzereingabe (M4)
e. Anzeigen einer weiteren interaktiven 3D-Szene (M5), aktualisiert in Bezug auf den Wundheilungsprozess, wobei die Aktualisierung auf der Auswertung der Benutzereingabe basiert, sodass der Benutzer (U) einen realistischen Wundheilungsprozess im Zeitraffer erleben kann.

2. Verfahren gemäß Anspruch 1, wobei das Bild einer Wunde ein 3D-Bild ist.

3. Verfahren gemäß Anspruch 1 oder 2, wobei das medizinische Szenario ferner umfasst: ein oder mehrere virtuelle Elemente, die anhand eines Patienten ausgewählt werden, der die Wunde zeigt, ein Krankenhausbett, eine Auswahl von Wundbehandlungsprodukten, eine Auswahl von Wundbehandlungsvorrichtungen und eine virtuelle Panoramaszene.

4. Verfahren gemäß einem oder mehreren der vorstehenden Ansprüche, wobei das Bild einer Wunde anhand von realen Patientendaten generiert wird.

5. Verfahren gemäß einem oder mehreren der vorstehenden Ansprüche, wobei das Bild der Wunde entweder anhand eines Einzelbildes einer realen Wunde generiert wird oder anhand mehrerer Bilder von realen Wunden generiert wird, beispielsweise durch maschinelles Lernen.

6. Verfahren gemäß einem oder mehreren der vorstehenden Ansprüche, wobei es sich bei den Wundbehandlungsoptionen um Wundbehandlungsprodukte handelt, insbesondere um Wundverbände oder Wundbehandlungsvorrichtungen.

7. Verfahren gemäß einem oder mehreren der vorstehenden Ansprüche, wobei der Benutzer (U) eine korrekte Ausführung der ein oder mehreren ausgewählten Wundbehandlungsoptionen in der VR-Umgebung beobachten kann.

8. Verfahren gemäß einem oder mehreren der vorstehenden Ansprüche, wobei der Wundheilungsprozess nach einem Zeitraum von 1 Minute bis 90 Tagen gezeigt wird, vorzugsweise nach einem Zeitraum von 1 Stunde bis 60 Tagen, noch bevorzugter nach einem Zeitraum von 1 Stunde bis 28 Tagen, insbesondere nach einem Zeitraum von 1 Stunde bis 7 Tagen oder nach einem Zeitraum von 1 Stunde bis 3 Tagen.

9. Verfahren gemäß einem oder mehreren der vorstehenden Ansprüche, wobei der Benutzer (U) zusätzliche Rückmeldungen oder Informationen zu der Behandlungsauswahl in der VR (Virtual Reality)-Umgebung erhält.

10. Verfahren gemäß einem oder mehreren der vorstehenden Ansprüche, wobei die Schritte a bis e wenigstens einmalig ausgeführt werden und wobei die Schritte b bis e wiederholt werden, bis eine Beendigungsbedingung erfüllt ist.

11. Verfahren gemäß einem oder mehreren der vorstehenden Ansprüche, wobei die erste interaktive 3D-Szene aus einer Mehrzahl von interaktiven 3D-Szenenoptionen ausgewählt wird, sei es durch eine Benutzerauswahl oder zufällig.

12. Technisches System (S1) für das Trainieren eines Benutzers (U), der eine Wundbehandlungsfachkraft ist, in einer VR (Virtual Reality)-Umgebung, umfassend eine VR (Virtual Reality)-Bildgebungseinheit (S2), wobei die VR (Virtual Reality)-Bildgebungseinheit (S2) eine kopfmontierte Anzeige ist, um ein 3D-Bild zu beziehen, wenigstens eine Eingabevorrichtung (S3), eine Einheit zum Erstellen virtueller Szenen (S4), eine Datenspeichereinheit und eine ausführbare Software, die dafür ausgelegt ist, die folgenden Schritte durchzuführen:
a. Anzeigen einer ersten interaktiven 3D-Szene (M1), die ein medizinisches Szenario darstellt, umfassend ein Bild einer Wunde, auf der VR (Virtual Reality)-Bildgebungseinheit (S2) mit Hilfe der Einheit zum Erstellen virtueller Szenen (S4)
b. Bereitstellen mehrerer Wundbehandlungsoptionen (M2), die auf der VR (Virtual Reality)-Bildgebungseinheit (S2) angezeigt werden und mit Hilfe der Eingabevorrichtung (S3) auswählbar sind, für den Benutzer (U)
c. Empfangen und Verarbeiten einer Benutzereingabe (U, M3), die einer oder mehreren Wundbehandlungsauswahlen entspricht, welche von dem Benutzer (U) durchgeführt werden, wobei jede Wundbehandlungsauswahl auf einer der Wundbehandlungsoptionen basiert, mit Hilfe der Einheit zum Erstellen virtueller Szenen (S4)
d. Auswerten der Benutzereingabe (M4) an der Einheit zum Erstellen virtueller Szenen (S4)
e. Anzeigen einer weiteren interaktiven 3D-Szene (M5), aktualisiert in Bezug auf den Wundheilungsprozess, wobei die Aktualisierung auf der Auswertung der Benutzereingabe basiert, auf der VR (Virtual Reality)-Bildgebungseinheit (S2) mit Hilfe der Einheit zum Erstellen virtueller Szenen (S4), sodass der Benutzer (U) einen realistischen Wundheilungsprozess im Zeitraffer erleben kann.

13. System (S1) gemäß Anspruch 12, wobei die Einheit zum Erstellen virtueller Szenen (S4) eine Rechenvorrichtung umfasst, umfassend eine oder mehrere Verarbeitungseinheiten, wie etwa eine CPU oder eine GPU.

14. System (S1) gemäß Anspruch 12 oder Anspruch 13, wobei jede der wenigstens einen Eingabevorrichtung(en) (S3) eine VR (Virtual Reality)-Steuerung ist, insbesondere eine drahtlose, handgehaltene, zweiteilige, 3D-Bewegungs-VR (Virtual Reality)-Steuerung, ein oder mehrere VR (Virtual Reality)-Handschuhe und/oder ein Ganzkörper-Lesesystem.

15. Computerprogramm, das die Ausführung des Verfahrens für das Trainieren eines Benutzers (U), der eine Wundbehandlungsfachkraft ist, in einer VR (Virtual Reality)-Umgebung ermöglicht, die in einem oder mehreren der Ansprüche 1 bis 11 vorgestellt wird.

16. Computerlesbare Datenspeichervorrichtung mit einem darauf vorliegenden Computerprogramm zum Ausführen des Verfahrens für das Trainieren eines Benutzers (U), der eine Wundbehandlungsfachkraft ist, in einer VR (Virtual Reality)-Umgebung, die in einem der Ansprüche 1 bis 11 vorgestellt wird.

## Revendications

1. Procédé mis en œuvre par ordinateur pour la formation d'un utilisateur (U), qui est un professionnel du soin des blessures, dans un environnement VR, le procédé comprenant les étapes suivantes :
a. afficher une première scène interactive 3D (M1) par les moyens techniques de la réalité virtuelle (VR), qui représente un scénario médical comprenant l'image d'une blessure, où les moyens techniques de la réalité virtuelle (VR) comprennent un visiocasque ;
b. fournir à l'utilisateur (U) de multiples options de traitement de blessure (M2) ;
c. recevoir et traiter une entrée utilisateur (M3) correspondant à une ou plusieurs sélections de traitement de blessure effectuées par l'utilisateur (U), chaque sélection de traitement de blessure étant basée sur l'une des options de traitement de blessure ;
d. évaluer l'entrée utilisateur (M4) ;
e. afficher une autre scène interactive 3D (M5) mise à jour en relation avec le processus de soin de blessure, la mise à jour étant basée sur l'évaluation de l'entrée utilisateur,
afin que l'utilisateur (U) puisse faire l'expérience d'un processus réaliste de soin de blessure en accéléré.

2. Procédé selon la revendication 1, dans lequel l'image d'une blessure est une image 3D.

3. Procédé selon la revendication 1 ou la revendication 2, dans lequel le scénario médical comprend en outre un ou plusieurs des éléments virtuels sélectionnés parmi un patient qui affiche la blessure, un lit d'hôpital, une sélection de produits de traitement des blessures, une sélection de dispositifs de traitement des blessures et une scène virtuelle panoramique.

4. Procédé selon une ou plusieurs des revendications précédentes, dans lequel l'image d'une blessure est générée à partir de données de patients réels.

5. Procédé selon une ou plusieurs des revendications précédentes, dans lequel l'image de la blessure est soit générée à partir d'une image unique d'une blessure réelle, soit générée à partir de plusieurs images de blessures réelles, par exemple au moyen d'un apprentissage automatique.

6. Procédé selon une ou plusieurs des revendications précédentes, dans lequel les options de traitement de blessure sont des produits de traitement de blessure, en particulier des pansements, ou des dispositifs de traitement de blessure.

7. Procédé selon une ou plusieurs des revendications précédentes, dans lequel l'utilisateur (U) peut observer une exécution correcte des une ou plusieurs options de traitement de blessure sélectionnées dans l'environnement VR.

8. Procédé selon une ou plusieurs des revendications précédentes, dans lequel le processus de guérison de blessure est montré après une période de 1 minute à 90 jours, de préférence après une période de 1 heure à 60 jours, plus préférentiellement après une période de 1 heure à 28 jours, en particulier après une période de 1 heure à 7 jours ou après une période de 1 heure à 3 jours.

9. Procédé selon une ou plusieurs des revendications précédentes, dans lequel l'utilisateur (U) reçoit un retour d'informations ou des informations supplémentaires concernant la sélection du traitement dans l'environnement de réalité virtuelle (VR).

10. Procédé selon une ou plusieurs des revendications précédentes, dans lequel les étapes a à e sont exécutées au moins une fois et dans lequel les étapes b à e sont répétées jusqu'à ce qu'une condition de terminaison soit satisfaite.

11. Procédé selon une ou plusieurs des revendications précédentes, dans lequel la première scène interactive 3D est sélectionnée parmi une pluralité d'options de scènes interactives 3D, soit par une sélection de l'utilisateur, soit de manière aléatoire.

12. Système technique (S1) pour la formation d'un utilisateur (U), qui est un professionnel du soin des blessures, dans un environnement de réalité virtuelle (VR), comprenant une unité d'imagerie de réalité virtuelle (VR) (S2), où l'unité d'imagerie de réalité virtuelle (VR) (S2) est un visiocasque, pour acquérir une image 3D, au moins un dispositif d'entrée (S3), une unité de construction de scène virtuelle (S4), une unité de stockage de données et un logiciel exécutable adapté pour exécuter les étapes suivantes :
a. afficher une première scène interactive 3D (M1), qui représente un scénario médical comprenant l'image d'une blessure, sur l'unité d'imagerie de réalité virtuelle (VR) (S2) au moyen de l'unité de construction de scène virtuelle (S4) ;
b. fournir à l'utilisateur (U) de multiples options de traitement de blessure (M2) affichées sur l'unité d'imagerie de réalité virtuelle (VR) (S2) et sélectionnables au moyen du dispositif d'entrée (S3) ;
c. recevoir et traiter une entrée utilisateur (U) (M3) correspondant à une ou plusieurs sélections de traitement de blessure effectuées par l'utilisateur (U), chaque sélection de traitement de blessure étant basée sur l'une des options de traitement de blessure au moyen de l'unité de construction de scène virtuelle (S4) ;
d. évaluer l'entrée utilisateur (M4) sur l'unité de construction de scène virtuelle (S4) ;
e. afficher une autre scène interactive 3D (M5) mise à jour en relation avec le processus de soin de blessure, la mise à jour étant basée sur l'évaluation de l'entrée utilisateur, sur l'unité d'imagerie de réalité virtuelle (VR) (S2) au moyen de l'unité de construction de scène virtuelle (S4),
afin que l'utilisateur (U) puisse faire l'expérience d'un processus réaliste de soin de blessure en accéléré.

13. Système (S1) selon la revendication 12, dans lequel l'unité de construction de scène virtuelle (S4) comprend un dispositif informatique comprenant une ou plusieurs unités de traitement, telles qu'une CPU ou une GPU.

14. Système (S1) selon la revendication 12 ou la revendication 13, dans lequel chacun de l'au moins un dispositif d'entrée (S3) est un contrôleur de réalité virtuelle (VR), en particulier un contrôleur de réalité virtuelle (VR) sans fil, portable, en deux parties, à mouvement 3D, un ou plusieurs gants de réalité virtuelle (VR) et/ou un système de lecture sur tout le corps.

15. Programme d'ordinateur permettant l'exécution du procédé de formation d'un utilisateur (U), qui est un professionnel du soin des blessures, dans un environnement de réalité virtuelle (VR) présenté dans une ou plusieurs des revendications 1 à 11.

16. Dispositif de stockage lisible par ordinateur dans lequel est intégré un programme informatique permettant d'exécuter le procédé de formation d'un utilisateur (U), qui est un professionnel du soin des blessures, dans un environnement de réalité virtuelle (VR) présenté dans l'une des revendications 1 à 11.
